# EUROPÄISCHE PATENTSCHRIFT

(11) **EP 0 631 489 B1**
(45) Veröffentlichungstag und Bekanntmachung des Hinweises auf die Patenterteilung: **15.12.1999**
(21) Anmeldenummer: 93906560.3
(22) Anmeldetag: 19.03.1993
(51) Int. Cl.: A61B 5/03, A61B 5/022

(54) **VERFAHREN UND VORRICHTUNG ZUM MESSEN VON VITALEN FETALEN PARAMETERN WÄHREND DER GEBURT**
PROCESS AND DEVICE FOR MEASURING FOETAL VITAL PARAMETERS DURING PARTURITION
PROCEDE ET DISPOSITIF DE MESURE DE PARAMETRES VITAUX DU FOETUS PENDANT L'ACCOUCHEMENT

(30) Priorität: 20.03.1992 DE 4209147
(43) Veröffentlichungstag der Anmeldung: 04.01.1995
(73) Patentinhaber: Rall, Gerhard, Dipl.-Ing., D-81545 München (DE); KNITZA, Reinhold, Dr., D-82131 Gauting (DE)
(72) Erfinder: Rall, Gerhard, Dipl.-Ing., D-81545 München (DE); KNITZA, Reinhold, Dr., D-82131 Gauting (DE)
(74) Vertreter: Grünecker, Kinkeldey, Stockmair & Schwanhäusser Anwaltssozietät
(86) Internationale Anmeldenummer: EP9300664
(87) Internationale Veröffentlichungsnummer: WO9318705

(56) Entgegenhaltungen:
- EP-A- 0 097 454
- EP-A- 0 135 840
- US-A- 4 476 871

## Beschreibung

Die Erfindung betrifft ein Verfahren und eine Vorrichtung zum Messen von vitalen fetalen Parametern während der Geburt.

In der Geburtshilfe wird bisher in der Praxis überwiegend eine Vorrichtung zum Durchführen der sogenannten Cardiotocographie (CTG) benutzt. Mit dieser Vorrichtung werden die kindliche Herzfrequenz und die mütterliche Wehentätigkeit in zwei Schreibspuren nebeneinander auf einem Registrierstreifen dargestellt, so daß z.B. eine Hebamme anhand dieser Aufzeichnungen den Geburtsvorgang überwachen kann.

Das CTG-Verfahren liefert allerdings keine unmittelbaren Parameter, vielmehr bedarf es einer Interpretation der Aufzeichnungen durch die Hebamme, die im Zweifel einen Arzt zuzieht.

Aus der US-A-4,476,871 ist eine Vorrichtung zum Erfassen von Parametern der Mutter während der Geburt beschrieben. Mit Hilfe eines zwischen dem führenden Teil des Feten und der Zervix einführbaren Drucksensor wird kontinuierlich die Erweiterung der Zervix gemessen. Ferner kann mit Hilfe von Elektroden am Drucksensor das EKG der Mutter erfaßt werden. Insgesamt eignet sich die Vorrichtung nur zum Erfassen von Parametern der Mutter.

Aus der EP 0 135 840 A2 ist ein Pulsoxymetriesensor bekannt, mit dem vitale Parameter des Feten während der Geburt erfaßt werden. Der Sensor wird mit Hilfe eines Vakuums am führenden Teil des Feten angesaugt und soll die Sauerstoffsättigung im Blut des Feten messen. Ferner können zusätzliche Einheiten zum Messen des Pulses und des EKG des Feten vorgesehen sein. Durch die Fixierung des Sensors mit Hilfe des Vakuums besteht die Gefahr, daß der Blutfluß des Feten in diesem Bereich beeinträchtigt wird und nur unzureichend genaue vitale Parameter erhalten werden.

Aufgabe der Erfindung ist es, ein Verfahren und eine Vorrichtung anzugeben, die absolute Werte von vitalen fetalen Parametern während der Geburt liefern, so daß Hebammen Meßwerte mit eindeutigen Aussagen erhalten.

Zur Lösung dieser Aufgabe sieht die Erfindung vor, daß der Blutdruck des Feten im arteriellen System diskontinuierlich gemessen wird, in dem mit Halte des Drucks zwischen dem ringförmigen uterinen Gewebes und dem führenden Teil des Feten und einem Drucksensor und einem die Blutflußcharakteristika erfassenden Sensor das Okklusionsverfehren durchgeführt wird.

Die Erfindung macht sich also das Prinzip der Okklusionsmessung des Blutdrucks zunutze.

Obwohl dieses Prinzip seit sehr langer Zeit allgemein bekannt ist, konnte die Fachwelt nicht erkennen, daß dieses Prinzip durch eine Weiterbildung überraschend auch für eine indirekte Blutdruckmessung des Feten während der Geburt herangezogen werden kann.

Bei dem bekannten Okklusionsverfahren zum indirekten und diskontinuierlichen Messen des Blutdrucks eines geborenen Menschen wird über dem Oberarm eine Manschette plaziert, die zu Beginn der Blutdruckmessung mit einer Luftpumpe so stark aufgeblasen wird, daß unter dem Druck der Manschette sämtliche Blutgefäße kollabieren und kein Blutfluß mehr erfolgt. Alsdann wird die Luft aus der Manschette langsam abgelassen. Dabei wird der interessierende Blutdruckbereich langsam durchfahren. Sobld der Manschettendruck leicht unter den systolischen Blutdruck gefallen ist, kann während der Zeit des höchsten arteriellen Blutdrucks der Systole wieder Blut in den Adern fließen.

Da die Adern noch weitgehend komprimiert sind, entsteht während des kurzen Moments, währenddessen arterielles Blut in den Arm einfließt, ein tauchendes Geräusch in den Arterieren jenseits der Manschette, das mittels eines Stethoskops abgehört werden kann. (Korotkow'sches Geräusch).

Das Geräusch kann selbstverständlich auch mittels eines Mikrofons erfaßt und z.B. elektronisch ausgewertet werden.

Im weiteren Verlauf der Messung wird der Manschettendruck immer weiter erniedrigt. Dabei werden die Korotkow'schen Geräusche immer lauter, dann aber wieder leiser. Sinkt der Manschettendruck schließlich unter den diastolischen Blutdruck, verschwinden die Korotkow'schen Geräusche ganz.

Der Manschettendruck, bei dem diese Geräusche erstmals auftreten, entspricht dem systolischen Blutdruck. Der Manschettendruck, bei dem diese Geräusche schließlich verschwinden oder an Intensität deutlich abnehmen, entspricht dem diastolischen Blutdruck.

Beim Verfahren gemäß der Erfindung wird auf überraschende Weise das ringförmige uterine Gewebe unmittelbar oder mittelbar zur Okklusionsmessung herangezogen.

So kann das Verfahren nach der Erfindung so ausgestaltet werden, daß das ringförmige uterine Gewebe während des Maximums einer Wehe, (Wehenakme) als Okklusionsmanschette gegenüber dem führenden Teil des Kindes verwendet wird, wobei der Okklusionsdruck (Druck in der Manschette höher als der systolische arterielle Blutdruck des Feten), zwischen dem uterinen Gewebe und dem führenden Teil des Kindes mittels eines Drucksensors und eines die Blutflußcharakteristika erfassenden Sensors ermittelt wird, wobei während des Nachlassens einer Wehe der systolische und der diastolische arterielle Blutdruck des Kindes bestimmit wird.

Ist die Wehe stark genug, wird der Blutfluß während des Maximums der Wehe (Wehenakme) im relevanten Teil des Feten vollständig unterbunden. Während des Nachlassens der Wehe können dann nacheinander der systolische und der diastolische Blutdruck des Feten bestimmt werden.

Ist die Wehe nicht so stark, so kann die Hebamme den notwendigen Okklusionsdruck durch Druck auf den Bauch bzw. auf die Gebärmutter der Kreißenden herbeiführen. In vielen Fällen mag es auch genügen, die Kreißende während einer Wehe zur Betätigung der Bauchpresse aufzufordern.

Es kann jedoch gemäß einer weiteren Ausgestaltung des Verfahrens gemäß der Erfindung das uterine Gewebe auch in der Weise herangezogen werden, daß zwischen dem uterinen Gewebe und dem führenden Teil des Feten ein mit einem Fluid gefüllter, elastischer Hohlring angeordnet wird, der bis zum Erreichen des Okklusionsdruckes aufgeblasen wird, wobei der systolische und der diastolische Blutdruck des Kindes während des Ablassens des Druckes im Hohlring mittels des Drucksensors und des die Blutflußcharakteristika erfassenden Sensors bestimmt wird.

So kann während einer nicht so starken Wehe der Hohlring zum Erreichen des erforderlichen Okklusionsdruckes zusätzlich aufgeblasen werden, so daß der okkludierende Druck den systolischen Druck des Feten erreicht.

Eine solche Messung des fetalen Blutdrucks im arteriellen Gefäßsystem kann aber auch von Wehen unabhängig mit Hilfe des aufblasbaren Hohlrings vorgenommen werden.

Erfindungsgemäß kann mit Hilfe einer am Fetus anbringbaren Elektrode das fetale EKG abgeleitet werden. Die dabei gewonnenen Signale können auch dazu dienen, die Erkennung der Blutflußcharakteristika zu erleichtern.

Das Verfahren der Erfindung kann auch kombiniert werden mit dem CTG-Verfahren und/oder dem EKG-Verfahren und/oder dem Verfahren der Hämoglobinometrie, d.h. vorzugsweise dem Verfahren der Pulsoximetrie, aber auch der Erfassung von Dyshämoglobinen und dem absoluten Hämoglobinwert.

Eine Vorrichtung zum Durchführen des Verfahrens kann gemäß der Erfindung so gestaltet sein, daß sie zum Durchführen des Okklusionsverfahrens geeignet ist, indem sie aus einem die Blutflußcharakteristika erfassenden Sensor, welcher dazu geeignet ist, an dem führenden Teil des Feten angeordnet zu werden, und einem Drucksensor, welcher dazu geeignet ist, zwischen dem uterinen Gewebe und dem Feten angeordnet zu werden, besteht.

Geeignete, auf Blutflußcharakteristika reagierende Sensoren sind bekannt, z.B. die entsprechenden Sensoren als Teile der Vorrichtungen gemäß der internationalen Publikation WO 90/01293 oder der DE-PS 38 10 008.

Auch z.B. Doppler-Effekt-Sensoren oder Piezofoliensensoren können dazu verwendet werden.

Der Drucksensor kann aus einem mit Fluid gefüllten, am vorderen Ende geschlossenen Schlauch bestehen, an dessen hinterem Ende ein Drucksensorelement angebracht ist.

Der Drucksensor kann aber auch als Ballonkatheter oder als intrauterine Drucksonde oder als ein sowohl den Okklusionsdruck als auch simultan den intrauterinen Druck erfassender doppellumiger Ballonkatheter ausgebildet sein.

Die Vorrichtung kann gemäß der Erfindung weiterhin in der Weise zum Durchführen des Okklusionsverfahrens geeignet sein, daß sie aus einem am führenden Teil des Feten angeordneten, die Blutflußcharakteristika erfassenden Sensor und aus einem zwischen dem ringförmigen uterinen Gewebe und dem führenden Teil des Feten angeordneten Drucksensor besteht, der als ein mit Fluid gefüllter, elastischer, aufblasbarer Hohlring, ausgebildet ist.

Gemäß der Erfindung können der Hohlring und der die Blutflußcharakteristika erfassende Sensor als Baueinheit gegeneinander fixiert sein.

Auf diese Weise können sie nicht gegeneinander verschoben werden, wenn z.B. der fetale Kopf nach dem Anlegen der Anordnung innerhalb der Gebärmutter wegrotiert.

Weitere Merkmale und Vorteile der Erfindung ergeben sich aus der nachfolgenden Beschreibung in Verbindung mit der Zeichnung, in welcher zeigen:
- Figur 1: im Schema ein erstes Beispiel einer Vorrichtung zum Durchführen der Verfahren,
- Figur 2: eine weitere solche Vorrichtung,
- Figur 3: ein anderes Beispiel einer solchen Vorrichtung,
- Figur 3a: eine Einzelheit zu Figur 3,
- Figur 4: eine weitere solche Vorrichtung,
- Figur 4a: eine Einzelheit zu Figur 4,
- Figur 5: noch ein anderes Beispiel einer solchen Vorrichtung,
- Figur 5a: eine Einzelheit zu Figur 5 und
- Figur 5b: eine weitere Einzelheit zu Figur 5a, wobei die Fig. 3a, 4a, 5a und 5b im vergrößernden Maßstab gezeichnet sind.

In den Ausführungsbeispielen ist mit 1 das uterine Gewebe, im nachfolgenden vereinfacht Muttermund genannt, und mit 1a die Scheide bezeichnet.

Gegen den Muttermund 1 wird während der Geburt der fetale Kopf 2 gepreßt.

Durch die Scheide 1a ist an fetalen Kopf 2 ein Element 3 eingebracht, das Teil eines fetalen Blutflußcharakteristika erfassenden Sensors 4 ist. Das Element kann z.B. eine zu diesem Zweck bereits verwendete Spirale, aber auch z.B. eine bekannte Piezofolie sein.

Der Blutflußsensor 4 liefert entsprechende Signale an eine (nicht gezeichnete) auswertende Einheit.

Um den Druck, der zwischen dem Muttermund 1 und dem Kopf 2 des Kindes herrscht und der okkludierend wirkt, erfassen zu können, ist noch eine weitere Vorrichtung vorgesehen.

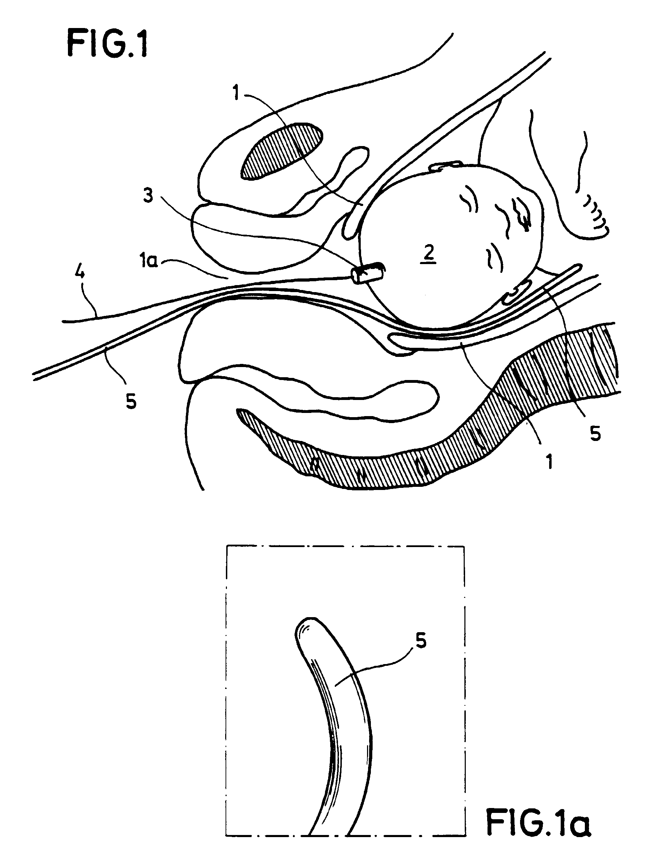

Im Beispiel nach Figur 1 wird diese Vorrichtung durch einen Schlauch 5 dargestellt, der zwischen dem Muttermund 1 und dem Kopf 2 des Kindes eingeführt ist.

Der Schlauch 5 ist am vorderen Ende geschlossen und mit einem Fluid gefüllt, das den zu messenden Druck an das hintere Ende des Schlauches 5 überträgt, wo ein Drucksensorelement angebracht ist.

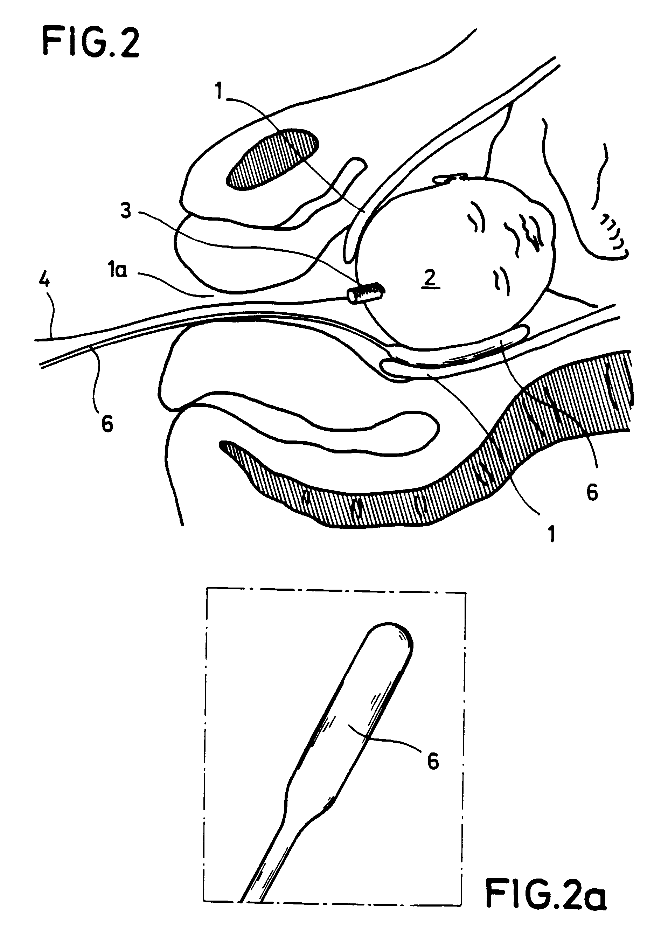

Im Beispiel nach Figur 2 ist als Sensor für die Erfassung des Okklusionsdruckes ein Ballonkatheter 6 in die Okklusionszone eingebracht.

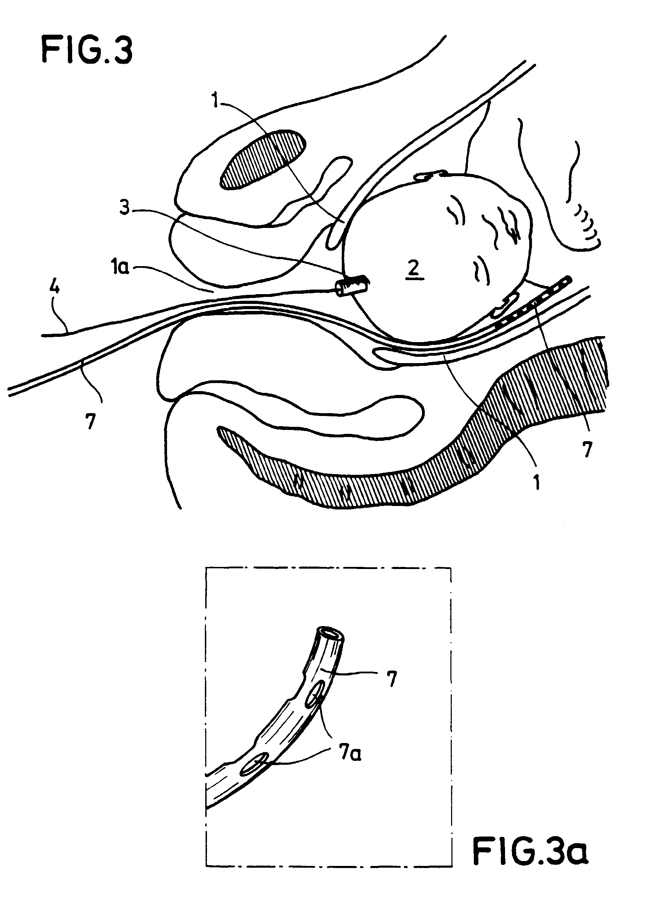

Eine weitere mögliche Ausführungsform des Sensors für die Okklusionsdruckerfassung ist die Benutzung einer bereits klinisch eingeführten intrauterinen Drucksonde 7. Etwaige Unterschiede zwischen dem intrauterinen Druck und dem Okklusionsdruck können über einen Umrechnungsfaktor ausgeglichen werden (Fig.3).

Gemäß Figur 3a sind an der Katheterspitze der intrauterinen Drucksonde 7 auf dem Umfang verteilte Öffnungen 7a angebracht.

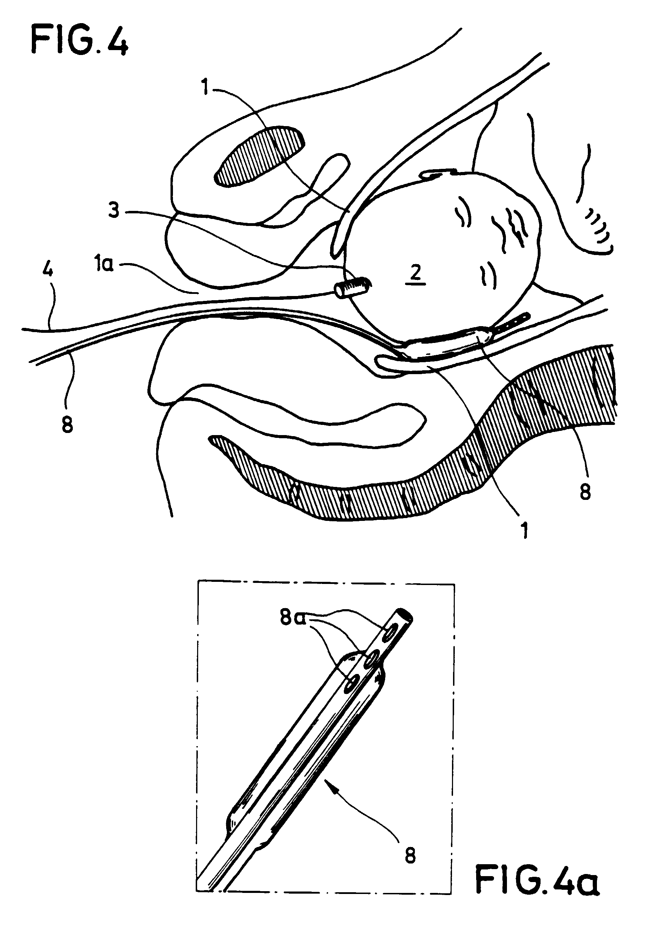

In Figur 4 ist der Sensor zum Erfassen des Okklusionsdruckes als doppellumiger Ballonkatheter 8 ausgebildet. (Der Schlauch des Katheters hat zwei voneinander getrennte Kanäle - Lumina -, wovon der eine Kanal mit dem Ballon des Katheters verbunden ist, der andere mit der Katheterspitze). Der Ballonkatheter ist sowohl zur Okklusionsdruckerfassung als auch zur simultanen Messung des intrauterinen Druckes einsetzbar.

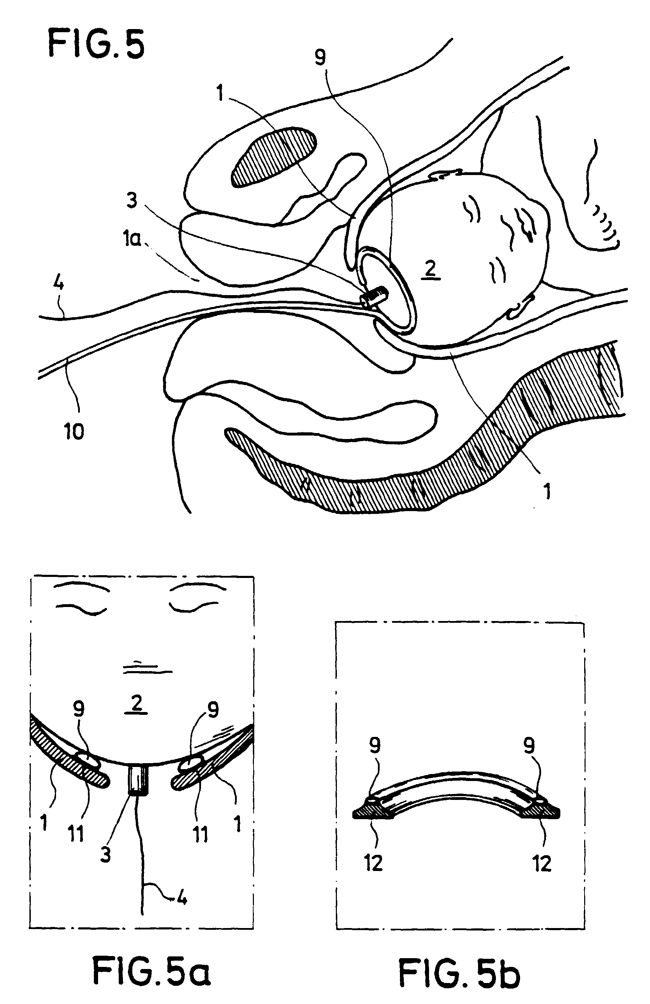

In Figur 4a ist ein solcher doppellumiger Ballonkatheter mit an der Katheterspitze seitlich angeordneten Öffnungen 8a schematisch gezeigt. Wenn der fetale Blutdruck im arteriellen Gefäßsystem bei schwächeren Wehen oder zwischen den Wehen gemessen werden soll, so kann dazu die Vorrichtung gemäß Fig. 5 dienen, um auch dann einen ausreichenden Okklusionsdruck zu erzielen.

Zu diesem Zweck ist ein mit einem Fluid gefüllter elastischer Hohlring 9 um das Blutflußsensorelement 3 herum zwischen dem Kopf 2 des Feten und dem uterinen Gewebe 1 eingebracht. Über einen Schlauch 10 kann der Hohlring 9 aufgeblasen und außerdem der in seinem Inneren herrschende Druck gemessen werden. Somit kann auch bei einer schwächeren Wehe der Okklusionsdruck durch zusätzliches Aufblasen des Hohlringes 9 erreicht werden. Auch zwischen den Wehen kann mit Hilfe des Hohlringes 9 ein ausreichender Okklusionsdruck erzielt werden. Der Hohlring 9 ist auf einer Seite abgeplattet, wobei diese Seite 11 verhältnismäßig hart ist und als Abstützung gegenüber dem uterinen Gewebe 1 dient.

Im Beispiel nach Figur 5b ist der Hohlring 9 auf einem flachen Abstützring 12 angebracht, der aus einem ausreichend festen aber flexiblen Werkstoff ist und sich am uterinen Gewebe 1 abstützt. Die Auflagefläche des Hohlringes 9 am Kopf 2 des Kindes ist kleiner als die Abstützfläche des Abstützringes 12.

So kann auf einfache Weise eine Druckvergrößerung erzielt werden. Der führende Teil des Feten muß nicht immer der Kopf sein. Auch der Steiß des Kindes ist dazu geeignet. Die Messung des fetalen Blutdrucks im arteriellen Gefäßsystem ist auch hier möglich.

Der Hohlring 9 bzw. der Abstützring 12 muß nicht endlos sein, er kann sich auch auf weniger als 360° erstrecken.

Das Verfahren kann auch vor dem Geburtsvorgang, z.B. nach einem vorzeitigen Blasensprung, also unabhängig von Wehen, durchgeführt werden.

Der systolische und diastolische fetale Blutdruck kann auch in umgekehrter Reihenfolge beim Anstieg einer Wehe gemessen werden. Dann kann während des Nachlassens einer Wehe die Messung zur Verdeutlichung nocheinmal in der üblichen Reihenfolge vorgenomen werden (Doppelbestimmung).

## Patentansprüche

1. Verfahren zum Messen von vitalen fetalen Parametern während der Geburt, wobei der Blutdruck des Feten im arteriellen System diskontinuierlich gemessen wird, indem mit Hilfe des Drucks zwischen dem ringförmigen uterinen Gewebe (1) und dem führenden Teil des Feten (2) und einen Drucksensor (6) sowie einem die Blutflußcharakteristika erfassenden Sensor (4) das Okklusionsverfahren durchgeführt wird.

2. Verfahren nach Anspruch 1, wobei ringförmiges uterines Gewebe (1) als Okklusionsmanschette gegenüber dem führenden Teil des Kindes (2) eingesetzt wird, wobei der Okklusionsdruck zwischen dem uterinen Gewebe (1) und dem führenden Teil des Kindes (2) mittels des Drucksensors (6) erfaßt wird und mittels des die Blutflußcharakteristika erkennenden Sensors (4) während des Nachlassens einer Wehe der systolische und der diastolische arterielle Blutdruck des Kindes (2) bestimmt wird.

3. Verfahren nach Anspruch 1, wobei zwischen dem ringförmigen uterinen Gewebe (1) und dem führenden Teil des Feten ein mit einem Fluid gefüllter, elastischer Hohlring (9) angeordnet wird, der bis zum Erreichen des Okklusionsdruckes aufgeblasen wird, wobei der systolische und der diastolische Blutdruck des Kindes während des Ablassens des Druckes im Hohlring (9) mittels des Drucksensors (6) und des die Blutflußcharakteristika erkennenden Sensors (4) bestimmt wird.

4. Verfahren nach Anspruch 1 oder 2, wobei von einem der Sensoren z.B. vom die Blutflußcharakteristika erfassenden Sensor (4) oder vom Drucksensor (6) das fetale EKG abgeleitet wird.

5. Verfahren nach einen der Ansprüche 1 bis 4, wobei gleichzeitig die fetale Hämoglobinometrie, vorzugsweise die fetale Pulsoximetrie und/oder das CTG-Verfahren und/oder das fetale EKG durchgeführt werden.

6. Vorrichtung zum Durchführen des Verfahrens nach Anspruch 1 oder 2, wobei die Vorrichtung zum Durchführen des Okklusionsverfahrens geeignet ist, indem sie aus einem die Blutflußcharakteristika erfassenden Sensor (4), welcher dazu geeignet ist, an dem führenden Teil (2) des Feten angeordnet zu werden, und einem Drucksensor (5, 6), welcher dazu geeignet ist, zwischen dem uterinen Gewebe (1) und dem Feten angeordnet zu werden, besteht.

7. Vorrichtung nach Anspruch 6, wobei der Drucksensor (5) aus einem mit Fluid gefüllten, am vorderen Ende geschlossenen Schlauch (5) besteht, an dessen hinterem Ende ein Drucksensorelement angebracht ist.

8. Vorrichtung nach Anspruch 6, wobei der Drucksensor als Ballonkatheter (6) ausgebildet ist.

9. Vorrichtung nach Anspruch 6, wobei der Drucksensor als klinisch bekannte intrauterine Drucksonde (7) ausgebildet ist.

10. Vorrichtung nach den Ansprüchen 6 und 9, wobei der Drucksensor als ein sowohl den Okklusionsdruck als auch simultan den intrauterinen Druck erfassender doppellumiger Ballonkatheter (8) ausgebildet ist.

11. Vorrichtung zum Durchführen des Verfahrens nach Anspruch 1 oder 3, wobei die Vorrichtung zum Durchführen des Okklusionsverfahrens geeignet ist, indem sie aus einem die Blutflußcharakteristika erfassenden Sensor (4), welcher dazu geeignet ist, am führenden Teil (2) des Feten angeordnet zu werden, und aus einem Drucksensor, welcher dazu geeignet ist, zwischen dem ringförmigen uterinen Gewebe (1) und dem führenden Teil (2) des Feten angeordnet zu werden, besteht, wobei der Drucksensor als ein mit Fluid gefüllter, elastischer, aufblasbarer Hohlring (9) ausgebildet ist.

12. Vorrichtung nach Anspruch 11, wobei der Hohlring (9) auf einer Seite zur Bildung einer Auflagefläche (11) am uterinen Gewebe (1) abgeplattet ist.

13. Vorrichtung nach Anspruch 12, wobei die abgeplattete Seite (11) aus einem steifen Werkstoff besteht.

14. Vorrichtung nach Anspruch 11, wobei der Hohlring (9) auf eine elastischen Abstützring (12) angeordnet ist, dessen am uterinen Gewebe (1) anliegende Fläche größer ist als die am führenden Teil des Feten anliegende Fläche des Hohlrings (9).

15. Vorrichtung nach Anspruch 11, wobei der elastische Hohlring (9) und der die Blutflußcharakteristika erfassende Sensor (4) als Baueinheit gegeneinander fixiert sind.

16. Vorrichtung nach Anspruch 6 oder 11 zum Durchführen des Verfahrens nach den Ansprüchen 1 und 4, wobei der die Blutflußcharakteristika erfassende Sensor (4) oder der Drucksensor (5, 6) mit elektrischen Kontakten zur Ableitung des kindlichen EKG versehen ist.

## Claims

1. A process for measuring vital fetal parameters during parturition, wherein the blood pressure of the fetus is discontinuously measured in the arterial system by carrying out the occlusion process by means of the pressure prevailing between the annular uterine tissue (1) and the leading part of the fetus (2) and by means of a pressure sensor (6) and a sensor (4) which senses the blood flow characteristics.

2. A process according to claim 1, wherein annular uterine tissue (1) is used as an occlusion cuff with respect to the leading part of the child (2), the occlusion pressure between the uterine tissue (1) and the leading part of the child (2) is sensed by means of the pressure sensor (6), and the systolic and the diastolic arterial blood pressure of the child (2) is determined during the subsiding of a contraction by means of the sensor (4) recognizing the blood flow characteristics.

3. A process according to claim 1, wherein an elastic hollow ring (9) filled with a fluid is diposed between the annular uterine tissue (1) and the leading part of the fetus, which ring (9) will be inflated until the occlusion pressure has been reached, the systolic and the diastolic blood pressure of the child is determined during the release of the pressure in the hollow ring (9) by means of the pressure sensor (6) and the sensor (4) recognizing the blood flow characteristics.

4. A process according to claim 1 or 2, wherein the fetal ECG is derived from one of the sensors, e.g. from the sensor (4) sensing the blood flow characteristics or from the pressure sensor (6).

5. A process according to any of claims 1 to 4, wherein the fetal hemoglobinometry, preferably the fetal pulse oximetry, and/or the CTG process and/or the fetal ECG are carried out at the same time.

6. A device for carrying out the process according to claim 1 or 2, wherein the device is suited for carrying out the occlusion process, by consisting of a sensor (4) suited to be disposed on the leading part (2) of the fetus and sensing the blood flow characteristics and of a pressure sensor (5, 6) suited to be disposed between the uterine tissue (1) and the fetus.

7. A device according to claim 6, wherein the pressure sensor (5) consists of a flexible tube (5) filled with fluid and closed at the front end, to whose rear end a pressure sensor element is affixed.

8. A device according to claim 6, wherein the pressure sensor is designed as a balloon catheter (6).

9. A device according to claim 6, wherein the pressure sensor is designed as a clinically known, intrauterine pressure probe (7).

10. A device according to claims 6 and 9, wherein the pressure sensor is designed as a double-lumen balloon catheter (8) detecting both the occlusion pressure and simultaneously the intrauterine pressure.

11. A device for carrying out the process according to claim 1 or 3 wherein the device is suited for carrying out the occlusion process, by consisting of a sensor (4) suited to be disposed on the leading part (2) of the fetus and sensing the blood flow characteristics, and of a pressure sensor suited to be disposed between the annular uterine tissue (1) and the leading part (2) of the fetus, said pressure sensor being designed as an elastic, inflatable hollow ring (9) filled with fluid.

12. A device according to claim 11, wherein the hollow ring (9) is flattened on one side to form a support surface (11) on the uterine tissue (1).

13. A device according to claim 12, wherein the flattened side (11) consists of a rigid material.

14. A device according to claim 11, wherein the hollow ring (9) is disposed on an elastic support ring (12) whose surface resting against the uterine tissue (1) is larger than the surface of the hollow ring (9) which rests against the leading part of the fetus.

15. A device according to claims 11, wherein the elastic hollow ring (9) and the sensor (4) sensing the blood flow characteristics are fixed relative to each other as a constructional unit.

16. A device for carrying out the process according to claim 6 or 11, wherein the sensor (4) sensing the blood flow characteristics or the pressure sensor (5, 6) is provided with electrical contacts for deriving the child's ECG.

## Revendications

1. Procédé pour mesurer des paramètres vitaux du foetus pendant l'accouchement, selon lequel on mesure la tension artérielle du foetus dans le système artériel de manière discontinue en réalisant l'occlusion à l'aide de la pression entre le tissu utérin annulaire (1) et la partie du foetus qui se présente (2) , et un capteur de pression (6) et un capteur (4) qui enregistre les caractéristiques de flux sanguin.

2. Procédé selon la revendication 1, selon lequel on utilise le tissu utérin annulaire (1) comme manchon d'occlusion par rapport à la partie de l'enfant qui se présente (2), la pression d'occlusion entre le tissu utérin (1) et la partie de l'enfant qui se présente (2) étant enregistrée à l'aide du capteur de pression (6), et la tension artérielle systolique et diastolique de l'enfant (2) étant définie pendant le relâchement d'une contraction à l'aide du capteur (4) qui identifie les caractéristiques de flux sanguin.

3. Procédé selon la revendication 1, selon lequel on place entre le tissu utérin annulaire (1) et la partie du foetus qui se présente un anneau creux élastique (9) qui est rempli d'un fluide et qui est gonflé jusqu'à ce que la pression d'occlusion soit atteinte, la tension artérielle systolique et diastolique de l'enfant étant définie pendant l'évacuation de la pression dans l'anneau creux (9) à l'aide du capteur de pression (6) et du capteur (4) qui identifie les caractéristiques de flux sanguin.

4. Procédé selon la revendication 1 ou 2, selon lequel l'électrocardiogramme est déduit de l'un des capteurs, par exemple du capteur (4) qui enregistre les caractéristiques de flux sanguin ou du capteur de pression (6).

5. Procédé selon l'une des revendications 1 à 4, selon lequel on effectue simultanément l'hémoglobinométrie du foetus, de préférence la pulsoxymétrie du foetus et/ou la cardiotocographie et/ou l'électrocardiogramme du foetus.

6. Dispositif pour mettre en oeuvre le procédé selon la revendication 1 ou 2, étant précisé que ce dispositif convient pour la mise en oeuvre du procédé d'occlusion, du fait qu'il se compose d'un capteur (4) qui enregistre les caractéristiques de flux sanguin et qui est apte à être disposé sur la partie du foetus qui se présente (2), et d'un capteur de pression (5, 6) qui est apte à être disposé entre le tissu utérin (1) et le foetus.

7. Dispositif selon la revendication 6, dans lequel le capteur de pression (5) se compose d'un tuyau (5) qui est fermé à son extrémité avant, qui est rempli de fluide et à l'extrémité arrière duquel est prévu un élément capteur de pression.

8. Dispositif selon la revendication 6, dans lequel le capteur de pression est conçu comme un cathéter à ballon (6) .

9. Dispositif selon la revendication 6, dans lequel le capteur de pression est conçu comme une sonde de pression intra-utérine (7) connue dans les hôpitaux.

10. Dispositif selon les revendications 6 et 9, dans lequel le capteur de pression est conçu comme un cathéter double à ballon (8) qui enregistre simultanément la pression d'occlusion et la pression intra-utérine.

11. Dispositif pour la mise en oeuvre du procédé selon la revendication 1 ou 3, étant précisé que ce dispositif convient pour mettre en oeuvre le procédé d'occlusion, du fait qu'il se compose d'un capteur (4) qui enregistre les caractéristiques de flux sanguin et qui est apte à être disposé sur la partie du foetus qui se présente (2), et d'un capteur de pression qui est apte à être disposé entre le tissu utérin annulaire (1) et la partie du foetus qui se présente (2), le capteur de pression étant conçu comme un anneau creux (9) élastique, gonflable et rempli de fluide.

12. Dispositif selon la revendication 11, dans lequel l'anneau creux (9) est aplati sur un côté pour former une surface d'appui (11) contre le tissu utérin (1).

13. Dispositif selon la revendication 12, dans lequel le côté aplati (11) se compose d'un matériau rigide.

14. Dispositif selon la revendication 11, dans lequel l'anneau creux (9) est disposé sur un anneau d'appui élastique (12) dont la surface appliquée contre le tissu utérin (1) est plus grande que la surface de l'anneau creux (9) appliquée contre la partie du foetus qui se présente.

15. Dispositif selon la revendication 11, dans lequel l'anneau creux élastique (9) et le capteur (4) qui enregistre les caractéristiques de flux sanguin sont fixés l'un par rapport à l'autre sous la forme d'une unité de construction.

16. Dispositif selon la revendication 6 ou 11 pour la mise en oeuvre du procédé selon les revendications 1 et 4, dans lequel le capteur (4) qui enregistre les caractéristiques de flux sanguin ou le capteur de pression (5, 6) est pourvu de contacts électriques destinés à déduire l'électrocardiogramme de l'enfant.
